# EUROPEAN PATENT APPLICATION

(11) **EP 3 824 842 A1**
(43) Date of publication of application: **26.05.2021**
(21) Application number: 19836934.0
(22) Date of filing: 16.07.2019
(51) Int. Cl.: A61C 5/90, A61B 1/24, A61B 13/00

(54) **LABIOLINGUAL RETRACTOR**

(30) Priority: 18.07.2018 BR 102018014627
(71) Applicant: Adriana Carlotto, Claudia, 69915-422 Rio Branco, AC (BR)
(72) Inventor: Adriana Carlotto, Claudia, 69915-422 Rio Branco, AC (BR)
(74) Representative: Zwicker, Jörk
(86) International application number: PCT/BR2019/050277
(87) International publication number: WO 2020/014761

(57) **Abstract**

The present invention relates to a lip and tongue retractor (100) comprising in a single central body (200) a central bulkhead (210) and a bridge (230). The central bulkhead (210) is provided with stabilizing rods (220), both resilient and movable between each other, the clearance thereof being maintained by a lock element (400) separated from the lip and tongue retractor (100); whereas the bridge (230) is equipped with a fitting end (231) for coupling to a convex body (300) separated from the central body (200).

## Description

### Field of application

The present invention pertains to the field of devices for surgical treatment of teeth, more specifically to tools and instruments for protecting soft tissues, teeth and parts of the mouth and expanding intraoral space.

### Introduction

The present invention relates to a lip and tongue retractor (or LTR), which allows for the removal of intraoral soft tissues, while providing wide mouth opening, thus resulting in relative isolation of the operative field in which the dental surgeon will intervene.

### Background of the Invention

It is possible to identify patent documents in this area developed over a hundred years ago, such as, for example, patent documents US 6,63507, US 3,406,452 and US 2,092,549, up to the present day as disclosed, for example, by the American patent document US 2004 126739.

However, many of such devices have flaws and limitations as discussed in more detail below, there being a remaining demand for devices which provide the dental professional with stability, reliability, ample visualization of the oral field and good interaction with other tools and instruments, whilst also providing the patient with safety and comfort during the surgical procedure, avoiding injuries and localized pain.

### State of the Art

Currently, devices for widening the intraoral operative field, as well as devices for protecting soft tissues are known in the prior art.

The prior art discloses, *inter alia,* rubber single block mouth openers (as well as the models sold by the company Indusbello® - more information available on the website http://www.ibindusbello.com.br/pt/produto/327/abritec-infantil) which must be fitted in crowns of upper and lower teeth, opposite to the intervened side so as to obtain a mouth opening.

However, this rubber block mouth opener does not remove nor protect the tongue or soft tissues, its fit in the crown of the teeth being only superficial, thus imparting reduced stability in the absence of one or more tooth. In addition, the stability thereof is compromised whenever unintended lateral movement and mouth opening movements occur.

Its shape and dimensions, together with the fact that it must be kept opposite to the side intended to be manipulated, reduce the operating space for the dentist to act in the oral cavity. There is also a potential risk of causing discomfort or pain in the patient, as it is kept in an unanesthetized area. And finally, in patients unable to collaborate, it is not always possible to obtain stability of the rubber block.

Another well-established device is the Molt mouth opener (as well as the models sold by the company Hu-Friedy® - more information available on the website http://www.hu-friedy.com/products/adult-molt-mouth-gag.html), the metal rods of which are separated by a ratchet-shaped gear. When fitted between dental arches opposite to the manipulated side, the rods provide a wide and stable mouth opening by locking the gear. Such a device can also be seen in patent document EP 0 396 525.

However, Molt's mouth opener does not separate nor protect intraoral soft tissues. The design of its rods results in active tips which are firmly accommodated in the palatal and sublingual mucosa. Similarly, the ratchet and the rods (which determine the opening of the device) are in close contact with the facial skin opposite to the anesthetized side, presenting a potential risk of discomfort and pain to the patient. Again, the potential for causing discomfort or pain is increased in cases of uncollaborative patients.

In addition, as a way to obtaining clearance from soft tissues, the market offers lip and tongue retractors. As disclosed by patent documents WO 2006 026129 and US 2013 230822, said type of retractor is commonly composed of a set of thin plastic rods, the shape of which results in a single piece. This arrangement provides for bilateral clearance of the upper and lower lips and of the jugal mucosa at the same time (with support on the rear teeth to maintain a slight mouth opening) and additionally the rods joined to the posterior and lower center of the oral cavity keep the tongue drawn backwards and downwards.

Here the provided mouth opening is also small and does not favor intraoral operative action. The thin rods are fragile and tend to destabilize the assembly when lateral movements of the dental arches and tongue occurs. Additionally, holding the tongue downwards and backwards offers a potential risk of choking sensation, gag reflex or both. Stability is compromised or is once again impossible to obtain when with patients uncollaborative with the procedure.

Finally, as in the above-mentioned American patent documents US 663,507, US 3,406,452, US 2,092,549 and US 2004 126739, there is the absolute isolation system for the total isolation of the operative field. Said system consists of a metal or plastic arch to which a rubber sheet is attached and extended. When pierced by a specific instrument, it is inserted through the dental crowns and sits accommodated on the gingival mucosa. However, for such to be achieved, it is also kept attached to the crowns of the teeth by means of metal clamps equally placed with a specific forceps. The diverse shapes and sizes of these clamps allow them to be chosen according to the crown of the tooth that will receive the aforementioned absolute isolation. In this manner, after the absolute isolation set is installed, the tongue and cheek mucosa is cleared by the extended rubber sheet, and mouth opening imposed by the handle of the metal clamp which stands as an obstacle to closing the mouth.

However, once again, it is possible to observe that the absolute isolation system also has flaws to be solved such as, for example, the fact that the absolute isolation system of the operative field through plastic or metallic arch sets, rubber dams and metal clamps is verifiably restricted to the procedures of operative dentistry and endodontics. Its use is limited or even prevented when it is impossible to obtain a clamp which fits the crown of the retaining tooth among those available on the market (for factors such as anatomy, eruption stage, destruction by tooth cavities or fracture).

The rubber dams, in turn, do not allow for direct visualization of the tissues below the isolation and the oropharynx. In addition, the use of absolute isolation is entirely related to the need of applying local anesthetics in the area in which the clamp will be placed, causing need for medication beyond the usual in cases in which local anesthesia would not be indicated for the treatment at hand.

Thus, it is noticeable that the above-mentioned objects and devices from the prior art do not yet allow for concomitant clearance and protection of soft tissues, wide mouth opening and freedom of movement for placing equipment and materials.

Additionally, they fail to provide all said advantageous characteristics, especially in view of patients who are not in a position to collaborate in keeping said devices stable such as, for example, children, elderly, sedated patients, or even patients who have some form of physical, intellectual and/or behavioral disability.

Therefore, it should be noted that there is a demand for a device of the aforementioned nature which will solve the problems of the prior art, especially a device that can be installed in the manipulated area, promoting wide mouth opening, wide and stable clearance and rigid protection of intraoral soft tissues, allowing free access to teeth and supporting tissues.

In addition, such a device must remain stable without risk of causing damage or pain in the patient, even in case of patients unable to collaborate with dental treatment, as is common among babies, children in emergency dental care, patients with disabilities, elderly patients, and hospitalized patients.

Finally, such a device as required by the state of the art should be easy to manipulate and allow for ample visualization of the oral field and good interaction with other tools and instruments, whilst providing the patient with safety and comfort during the surgical procedure, avoiding injuries and localized pain.

### Objectives of the Invention

One of the objectives of the present invention is, therefore, to provide a lip and tongue retractor comprising in one central body a central bulkhead and a bridge, the central bulkhead being provided with stabilizing rods, both resilient and movable between each other, the clearance distance thereof maintained by a lock element separated from the central body; and the bridge provided with a fitting end for coupling to a convex body separated from the central body.

Other features and details of the features are represented by the dependent claims on the attached set of claims.

### Brief description of the drawings

For a better understanding and visualization of the subject matter, the present invention will now be described with reference to the attached figures, representing the obtained technical effect through an example embodiment without limiting the scope of the present invention, in which:

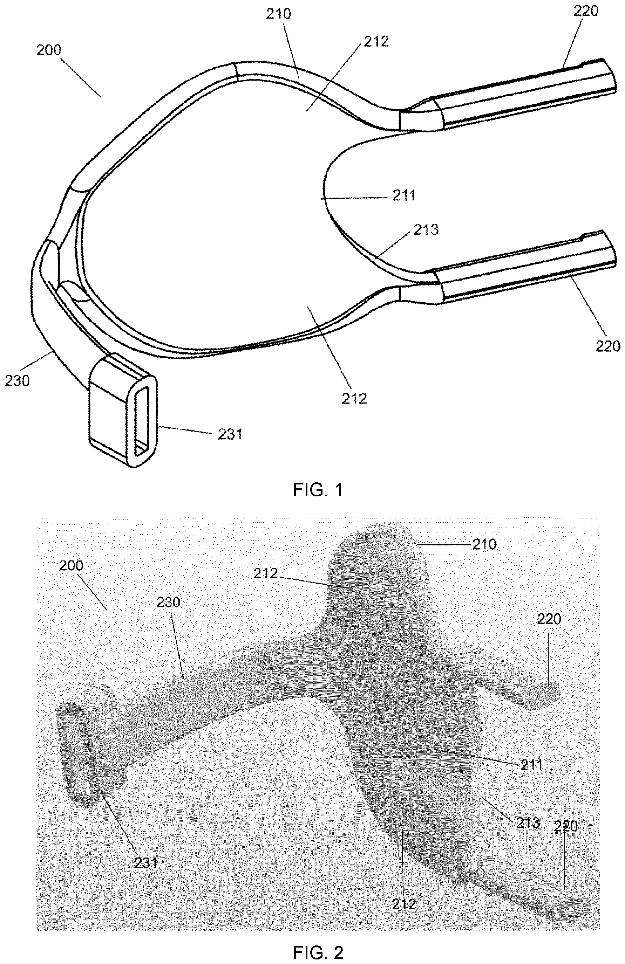

- Figure 1:: schematically shows a perspective view of the central body of a lip and tongue retractor according to the invention;
- Figure 2:: shows a perspective view of the central bulkhead of the lip and tongue retractor from Figure 1;

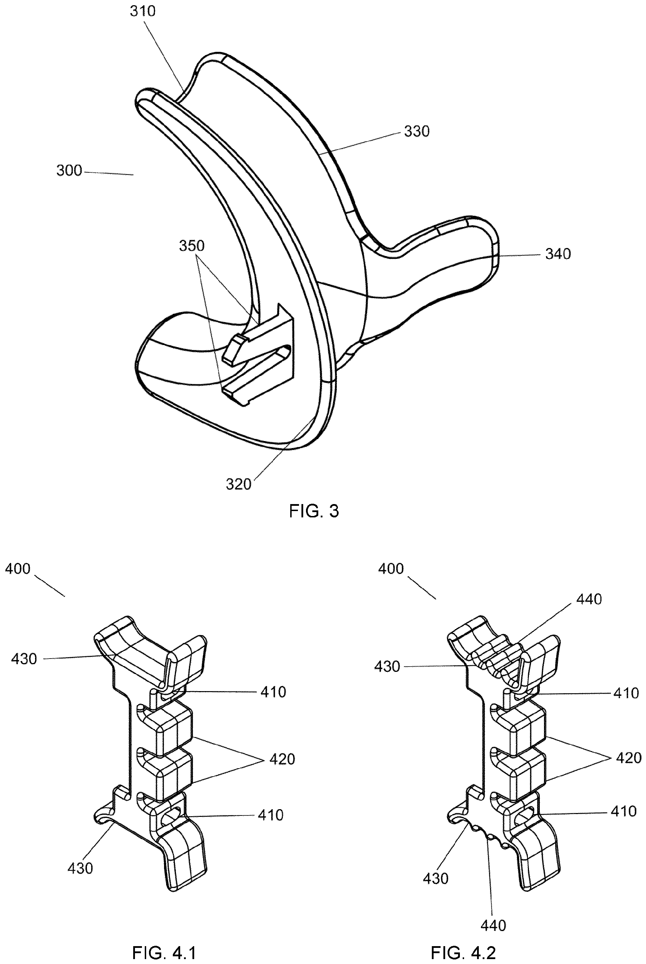

- Figure 3:: schematically shows a perspective view of the convex body of the lip and tongue retractor;
- Figure 4.1:: schematically shows a perspective view of a lock element of the lip and tongue retractor according to the invention;
- Figure 4.2:: schematically shows a perspective view of another lock element of the lip and tongue retractor according to the invention;

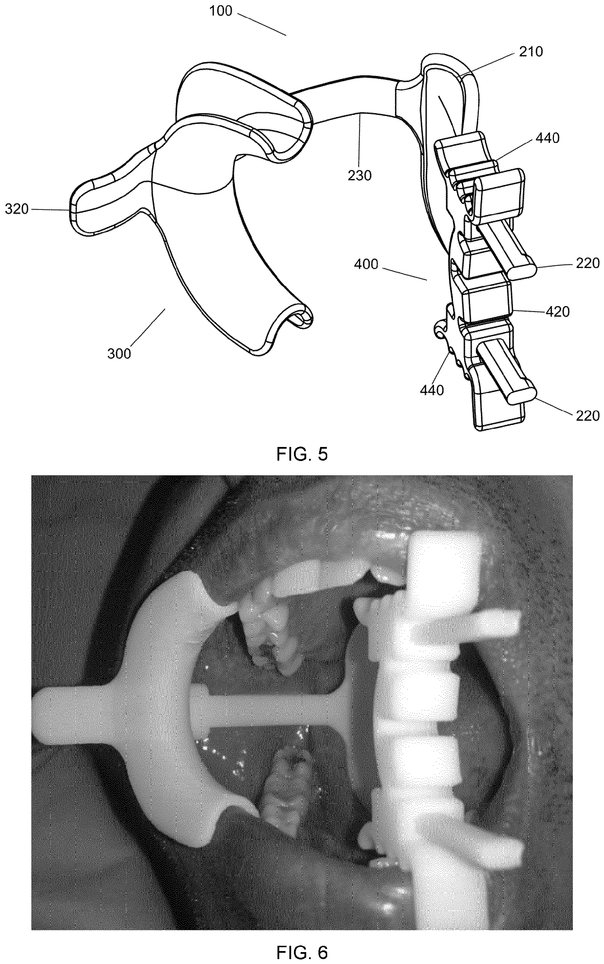

- Figure 5:: schematically shows a perspective view of the lip and tongue retractor, with the lock element and the convex body coupled to the central body; and
- Figure 6:: shows a view of the lip and tongue retractor according to the invention whilst in use arranged inside an oral cavity.

### Detailed Description of the Invention

The present invention will now be described in detail based on the aforementioned figures, but without being limited to them.

The lip and tongue retractor (100), according to the invention, basically comprises:
(i) A central body (200) equipped with
   - central bulkhead (210),
   - stabilizing rods (220),
   - bridge (230), and
   - fitting end (231);
(ii) A convex body (300) equipped with
   - groove (310),
   - larger wall (320),
   - smaller wall (330),
   - flap (340), and
   - locks (350);
(iii) A lock element (400) provided with
   - slots (410),
   - indents (420), and
   - bases (430).

The central bulkhead (210), stabilizing rods (220), bridge (230) and fitting end (231) elements are formed as a single piece, made from surgically suitable materials, such as, but not limited to, resilient, non-toxic, autoclavable materials, and can also be translucent/transparent, monochromatic, polychromatic, colored, having drawings or appliques, *inter alia.*

The central bulkhead (210) leads to stabilizing rods (220), resilient and movable between each other, straight at their ends, the clearance thereof maintained by the lock element (400) separated from the central body (200). Note that the stabilizing rods (220) by being resilient and movable between each other allow for contraction, enabling the introduction of lip and tongue retractor (100) in the patient's oral cavity. The central bulkhead (210) forms a central arch (213), in the space generated by stabilizing rods (220), in addition to having a central groove (211) and protrusions (212). Since the central bulkhead (210) is convex to the patient's pterygomandibular raphe, the wall formed by the central groove (211) and protrusions (212) acts as a means for retaining and isolating the patient's tongue in an ergonomic and comfortable manner.

The central body (200) of the lip and tongue retractor (100) also has a bridge (230) which is provided with a fitting end (231) for coupling to the convex body (300) separated from the central body (200).

Said bridge (230) extends to the pterygopalatine commissure and unites with the central bulkhead (210) and, together with the convex body (300), is responsible for the clearance of the lips and adjacent cheek mucosa from the dental professional's field of action.

The bridge (230) has its rear part facing the interior of the oral cavity immediately above and behind the retromolar triangle and abuts the pterygomandibular raphe. Note that the rear portion of the bridge (230) is evidently convex in relation to the pterygomandibular raphe when in use.

The convex body (300) is coupled by a reversible fit to the fitting end (231) of the bridge (230), so as to have its groove (310) facing the opening of the patient's mouth, having a shape that adapts anatomically to the patient's labial commissure and adjacencies thereof, providing protection to tissues and clearance for access to the oral cavity by isolating the labial commissure. For handling the convex body (300) and the lip and tongue retractor (100) as a whole, the convex body (300) has a flap (340) in the middle region of the smaller wall (330). For joining the convex body (300) with the central body (200), the convex body (300) is provided with locks (350) in the middle region of its larger wall (320) to engage with the fitting end (231) of the bridge (230).

Once installed, the lip and tongue retractor (100) provide gradual clearance of the arches, the module of which is made possible and maintained by the lock element (400), provided with two slots (410) into which the stabilizing rods (220) of the central bulkhead (210) fit, thus maintaining the lip and tongue retractor (100) stable. The lock element (400) is also provided with indents (420) in its central portion, and bases (430) in its upper and lower portions to accommodate the incisal/occlusal faces of the patient's upper and lower teeth. The bases (430) of the lock element (400) may also be provided with grooves (440) for a better accommodation of the patient's teeth when the lip and tongue retractor is in use.

The lock element (400) can be manufactured in different sizes (lengths) allowing for a greater or lesser range of mouth opening. When fitted, the lock element (400) provides a firm separation from the dental arches and, when detached, allows for easy removal of the lip and tongue retractor (100).

The indents (420) of the lock element (400) allow for it to be adaptable to different mouth opening amplitudes. When the curvature of the lock element (400) is contracted joining the indents (420), greater mouth opening amplitude is achieved. When the indents (420) are removed, thus generating a convex arch in relation to the lips, less mouth opening amplitude is achieved. This allows for the lip and tongue retractor (100) to have greater adaptability, thus being more suitable for patients unable to collaborate with the procedure.

One skilled in the art may also understand that the lock element (400) is resilient and made of surgically suitable, non-toxic and autoclavable material.

Thus, the use of lip and tongue retractor (100) according to the invention and according to the embodiment illustrated by the attached drawings simultaneously solves several problems of the prior art.

The lip and tongue retractor (100) therefore allows for the simultaneous clearance and protection of soft tissues, in addition to allowing for interaction with other known tools and devices, as the visualization of intraoral space and the reach of such tools and devices are improved.

The present lip and tongue retractor (100) solves the serious problem of lack of stability in situations with patients unable to collaborate with the procedure. As illustrated in the figures, the suitability provided by the positioning of the lock element (400) allows for the lip and tongue retractor (100) to be firmly attached whilst not harming the patient during treatment, thus eliminating the risk or disadvantage of subsequent pain or discomfort. The firm fit of the lip and tongue retractor (100) also foregoes additional stabilization provided by a health professional's hand the during the procedure, allowing said professional to have both hands free to easily perform the procedure, as well as handling other tools.

In addition, the multiplicity of positions provided by the present object also allows for adaptation to different dental arch configurations.

As discussed above, one of the problems found in prior art is the adaptation to arches in which teeth are missing (other devices and systems are destabilized in the absence of some teeth) or even upon movement by the patient.

Both difficulties are overcome by the lip and tongue retractor (100) of the present application, as the bases (430) of the lock element (400) are supported by the incisal/occlusal faces of the dental elements or on the edentulous alveolar ridge of the opposite arch, and the same lock element (400) provides stiffness to the chosen opening.

Another evident advantage of lip and tongue retractor (100) is its ambidextrous feature, i.e., the possibility of being used both for the right side and the left side of the mouth.

Additionally, again regarding patient comfort, the present lip and tongue retractor (100) does not hold the patient's tongue downwards and backwards. This configuration avoids the potential risk of a choking sensation, geg reflex or both, as generated by devices such as those comprised in prior art.

Finally, it should be noted that the present lip and tongue retractor (100) is suitable for several types of mouth-related procedures and is not limited only to dental surgical procedures (for example and illustrative purposes only, aesthetic procedures).

### Conclusion

It will be easily understood by those skilled in the art that modifications can be made to the present invention without departing from the concepts set out in the description above. Such modifications should be considered as included within the scope of the present invention. Consequently, the particular embodiments described in detail above are only illustrative and exemplary, and do not limit the scope of the present invention, which should be given the full extent of the appended claims and any and all equivalents thereof.

## Claims

1. Lip and tongue retractor (100) **characterized in that** it comprises, in a single central body (200), a central bulkhead (210) and a bridge (230).

2. Lip and tongue retractor (100) according to claim 1, **characterized in that** the central bulkhead (210) is provided with stabilizing rods (220), both resilient and movable between each other, the clearance distance thereof maintained by a lock element (400) separated from the central body (200).

3. Lip and tongue retractor (100) according to claim 1, **characterized in that** the bridge (230) is provided with a fitting end (231) for coupling to a convex body (300) separated from the central body (200).

4. Lip and tongue retractor according to claim 1, **characterized in that** the central bulkhead (210) is provided with a central groove (211) and protrusions (212) on the sides of said central groove.

5. Lip and tongue retractor according to claim 1, **characterized in that** the central bulkhead (210) forms a central arch (213) in the space generated by the stabilizing rods (220).

6. Lip and tongue retractor according to claim 1, **characterized in that** the rear portion of the central bulkhead (210) faces the interior of the oral cavity proximal to the pterygomandibular raphe.

7. Lip and tongue retractor according to claim 1, **characterized in that** the lock element (400) separated from the central body (200) is provided with slots (410) for fitting with the stabilizing rods (220) of the central bulkhead (210), indents (420) in its central portion, and bases (430) in its upper and lower portions to accommodate the incisal/occlusal faces of the upper and lower teeth.

8. Lip and tongue retractor according to claim 1, **characterized in that** the lock element (400) may be provided with grooves (440) in its bases (430).

9. Lip and tongue retractor according to claim 1, **characterized in that** the convex body (300) is provided with a larger wall (320), a smaller wall (330), a flap (340) in the medial region of the smaller wall (330), and locks (350), in the middle region of the larger wall (320), for reversible fitting to the fitting end (231) of the bridge (230).
